(19) Europäisches Patentamt

European Patent Office

Office européen des brevets

(11) **EP 4 691 348 A1**

(12) **EUROPEAN PATENT APPLICATION**
published in accordance with Art. 153(4) EPC

(43) Date of publication:
**11.02.2026  Bulletin 2026/07**

(21) Application number: **24823564.0**

(22) Date of filing: **25.04.2024**

(51) International Patent Classification (IPC):
*A61B 5/00* (2006.01)

(52) Cooperative Patent Classification (CPC):
**A61B 5/00**

(86) International application number:
**PCT/KR2024/005630**

(87) International publication number:
**WO 2024/258043 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.06.2023  KR 20230075440
07.08.2023  KR 20230102934**

(71) Applicant: Samsung Electronics Co., Ltd.
**Suwon-si, Gyeonggi-do 16677 (KR)**

(72) Inventors:
• **KIM, Younghyun
  Suwon-si, Gyeonggi-do 16677 (KR)**
• **PARK, Minho
  Suwon-si, Gyeonggi-do 16677 (KR)**
• **AHN, Joongwoo
  Suwon-si, Gyeonggi-do 16677 (KR)**
• **YUN, Inho
  Suwon-si, Gyeonggi-do 16677 (KR)**

(74) Representative: **Appleyard Lees IP LLP
15 Clare Road
Halifax HX1 2HY (GB)**

(54) **OPTICAL SENSOR AND ELECTRONIC DEVICE INCLUDING SAME**

(57)     An electronic device includes: a light-emitting unit disposed on a substrate, where the light-emitting unit includes light sources configured to emit light in different wavebands; first optical waveguides configured to transfer light emitted through the light-emitting unit in a first direction parallel to a surface of the substrate; and a light concentration change member configured to change an angle of light such that light transferred from the first optical waveguides is discharged in a second direction substantially perpendicular to the first direction by the light concentration change member, where the light concentration change member has a structure configured to concentrate, at one point, light rays transferred from the first optical waveguides in different positions.

FIG. 2

211   213   210   217

Optical sensor

| Light-emitting unit | Light concentration change member | Light-receiving unit |

**EP 4 691 348 A1**

**Description**

**[Technical Field]**

The disclosure relates to an optical sensor and an electronic device including the same.

**[Background Art]**

Electronic devices may provide various functions or services to users, based on information collected through sensors. For example, an electronic device may include an optical sensor including at least one light-emitting element and light-receiving element. The optical sensor may measure the electronic device user's biometric information by using light in a specific waveband. The biometric information may include, for example, saturation of percutaneous oxygen (SpO2), heart rate (HR), photoplethysmography (PPG), galvanic skin response (GSR), electrocardiography (ECG), and bioelectrical impedance.

The above information may be provided as related art to aid understanding of the present disclosure. No claim or decision is made as to whether any of the foregoing can be applied as prior art related to this disclosure.

**[Disclosure of Invention]**

**[Technical Problem]**

Optical sensors use one or more wavelengths and light sources to measure various components of biometric information, and the waveband of required light may differ depending on the biometric measurement information. Compact electronic devices use light sources that emit light in specific wavebands only, and a large number of light sources may thus be necessary to measure various pieces of biometric information. However, if the number of light sources increases, light is emitted in different positions and thus reach the biometric measurement subject (for example, skin) in different positions, depending on the waveband, thereby generating a difference in the path of light. In addition, in the case of a living body, which has a difference in tissue component, it may be difficult to acquire reliable data if there is a difference in the path of light with regard to each waveband.

Accordingly, an optical sensor (for example, a biometric sensor) requiring multiple wavebands or multiple light sources needs a structure for concentrating rays of light radiated in positions in which respective light sources are disposed into a single ray. However, conventional light-concentrating structures (for example, prisms, echelle gratings, arrayed waveguide gratings (AWG), or angled multi-mode interferometers (AMMI)) have a large volume and thus are not appropriate for compact electronic devices. Therefore, there is a need for a new structure appropriate for compact electronic devices.

Solution to Problem

Various embodiments may propose an optical sensor appropriate for a compact electronic device and an electronic device including the same.

However, problems to be solved by the disclosure are not limited to the above-mentioned problems, and may be variously expanded without deviating from the idea and scope of the disclosure.

[Technical Solution]

According to an embodiment an optical sensor may include a light-emitting unit disposed on a substrate, where the light-emitting unit includes light sources configured to emit light in different wavebands. According to an embodiment an optical sensor may include first optical waveguides configured to transfer light emitted through the light-emitting unit in a first direction parallel to a surface of the substrate. According to an embodiment an optical sensor may include a light concentration change member configured to change an angle of light such that light transferred from the first optical waveguides is discharged in a second direction substantially perpendicular to the first direction by the light concentration change member. According to an embodiment the light concentration change member may has a structure configured to concentrate, at one point, light rays transferred from the first optical waveguides in different positions.

According to an embodiment, an electronic device may include an optical sensor.

According to an embodiment an electronic device may include a light-emitting unit disposed on a substrate, where the light-emitting unit includes light sources configured to emit light in different wavebands. According to an embodiment an electronic device may include first optical waveguides configured to transfer light emitted through the light-emitting unit in a first direction parallel to a surface of the substrate. According to an embodiment an electronic device may include a light concentration change member configured to change an angle of light such that light transferred from the first optical waveguides is discharged in a second direction substantially perpendicular to the first direction by the light concentration change member. According to an embodiment the light concentration change member may has a structure configured to concentrate, at one point, light rays transferred from the first optical waveguides in different positions.

[Advantageous Effects of Invention]

An optical sensor and an electronic device according to various embodiments are advantageous in that a light concentration change member having a simple structure applied to the electronic device instead of light concentration devices having a complicated structure and a large volume (for example, echelle gratings, arrayed waveguide gratings (AWG), angled multi-mode interferometers (AMMI)) may be applied to the optical sensor, thereby contributing to process simplification and cost saving. An optical sensor and an electronic device according to an embodiment may simplify the structure of the optical sensor such that, even if a large number of light sources are mounted to measure various components of biometric information, the electronic device can be made compact.

Various other advantageous effects identified explicitly or implicitly through the disclosure may be provided.

[Brief Description of Drawings]

FIG. 1 is a block diagram illustrating an example electronic device in a network environment 100 according to various embodiments.
FIG. 2 illustrates a schematic configuration of a biometric sensor included in an electronic device according to an embodiment.
FIG. 3A and FIG. 3B illustrate examples of the structure of an optical sensor included in an electronic device according to an embodiment.
FIG. 4A illustrates an example of the structure of an optical sensor included in an electronic device according to an embodiment.
FIG. 4B and FIG. 4C illustrate the structure of a grating coupler according to an embodiment.
FIG. 5 illustrates an example of the structure of an optical sensor included in an electronic device according to an embodiment.
FIG. 6 illustrates an example of the structure of an optical sensor included in an electronic device according to an embodiment.
FIG. 7A and FIG. 7B illustrate examples of the structure of an optical sensor included in an electronic device according to an embodiment.
FIG. 8A and FIG. 8B illustrate examples of the structure of an optical sensor included in an electronic device according to an embodiment.
FIG. 9A to FIG. 9C illustrate examples of the structure of an optical sensor included in an electronic device according to an embodiment.
FIG. 10A to FIG. 10C illustrate examples of the structure of an optical sensor included in an electronic device according to an embodiment.
FIG. 11A to FIG. 11C illustrate examples of the structure of an optical sensor included in an electronic device according to an embodiment.
FIG. 12 illustrates an example of the structure of an optical sensor included in an electronic device according to an embodiment.
FIG. 13A to FIG. 13C illustrate examples of a lens structure included in an electronic device according to an embodiment.
FIG. 14A to FIG. 14C illustrate examples of a lens structure included in an optical sensor according to an embodiment.

[Mode for the Invention]

Hereinafter, with reference to the drawings, embodiments of the present disclosure will be described in detail so that those skilled in the art can easily practice them. However, the present disclosure may be implemented in many different forms and is not limited to the embodiments described herein. In relation to the description of the drawings, identical or similar reference numerals may be used for identical or similar components. Additionally, in the drawings and related descriptions, descriptions of well-known functions and configurations may be omitted for clarity and brevity.

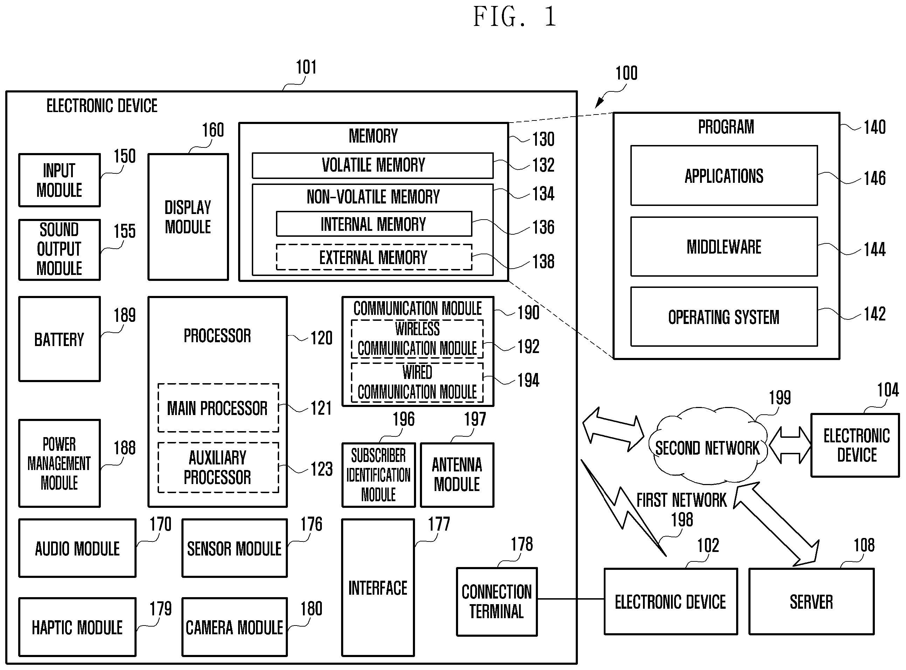

FIG. 1 is a block diagram illustrating an example electronic device in a network environment 100 according to various embodiments.

Referring to Fig. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, and/or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and/or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semisupervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof, but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 and/or the non-volatile memory 134. The non-volatile memory 134 may include an internal memory 136 and/or an external memory 138.

The program 140 may be stored in the memory 130 as software, and may include, for example, an operating system (OS) 142, middleware 144, and/or an application 146..

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high-definition multimedia interface (HDMI), a universal serial bus (USB) interface, a secure digital (SD) card interface, or an audio interface.

The connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, and/or flashes.

The power management module 188 may manage power supplied to and/or used by the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, a fuel cell, or a combination thereof.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a millimeter (mm) Wave antenna module. According to an embodiment, the mmWave antenna module may include a printed circuit board, a radio frequency integrated circuit (RFIC) disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, a home appliance, or the like. According to an embodiment of the disclosure, the electronic devices are not limited to those described above..

An electronic device 101 according to an embodiment may include a biometric sensor or an optical sensor capable of various pieces of biometric information. The biometric sensor or optical sensor disclosed herein may be applied not only to portable devices and wearable devices, but also to medical devices used in hospitals or examination institutions, personally held small medical devices, or health care devices.

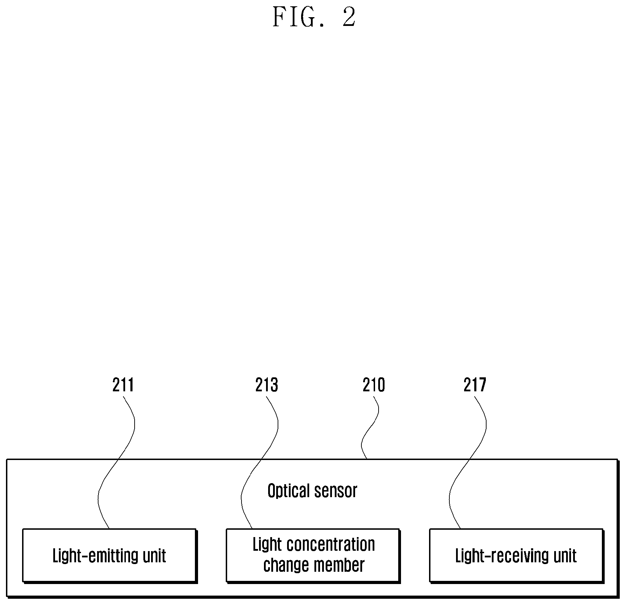

FIG. 2 illustrates a schematic configuration of a biometric sensor included in an electronic device according to an embodiment.

Referring to FIG. 2, the biometric sensor or optical sensor (for example, the sensor module 176 in FIG. 1) 210 included in an electronic device according to an embodiment (for example, the electronic device 101 in FIG. 1) may include a light-emitting unit 211, a light concentration change member 213, and a light-receiving unit 217. The optical sensor 210 may be operatively connected to the processor 120 in FIG. 1.

In an embodiment, the hardware of the electronic device 101 is operatively coupled such that the second hardware is controlled by the first hardware, such that a direct connection or an indirect connection between the hardware is wired, alternatively, it may mean established wirelessly.

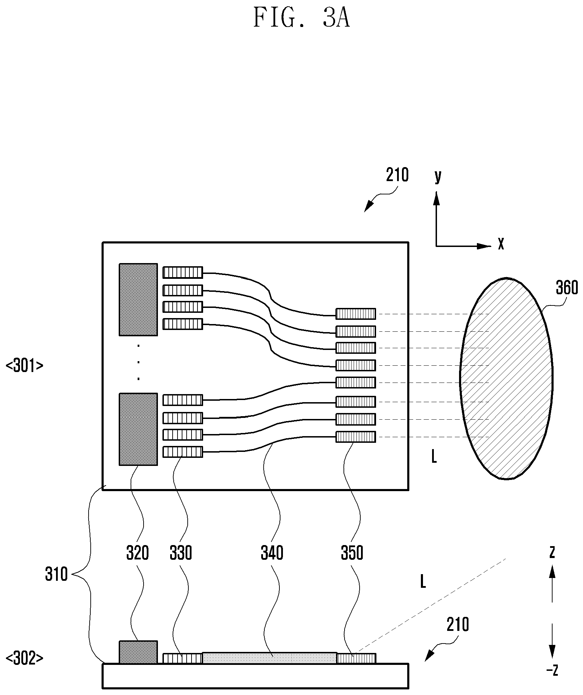

According to an embodiment, the light-emitting unit (or light-emitting array) 211 may be designed to generate optical signals in multiple colors (or multiple wavelengths) or in a broadband (or various wavelengths) in order to measure multiple pieces of biometric information. The light-emitting unit 211 may include one or more light sources. The light source may include a laser diode (LD) configured to generate light, a light-emitting diode (LED), and/or other light sources. For example, the light source may be designed in a vertical cavity surface emitting laser (VCSEL) type so as to radiate light in the vertical direction (for example, along z-axis in FIGS. 3A to 7A), may have an edge-emitting laser coupled to a reflecting mirror, or may have an edge-emitting laser chip attached in the vertical direction, but is not limited thereto.

As an example, the light source may include multiple luminous sources/luminous elements (for example, a first luminous element, a second luminous element, ..., an $N^{th}$ luminous element) so as to emit light in different wavebands. The luminous elements may be the same kind of light sources and/or different kinds of light sources. In the case of the same kind of light sources, the luminous elements may be designed to emit light in different wavebands.

According to an embodiment, the light-emitting unit (or light-emitting array) 211 may include a filter structure (or a filter array) (not illustrated) configured to filter light emitted from the light source into a specific waveband. The filter structure may include filters configured to selectively transmit necessary light only according to biometric measurement information. For example, in the case of SpO2 measurement, filters may be designed to transmit an approximately 640 nm waveband and an approximately 940 nm waveband only. This is only an example, and the filters may vary depending on the biometric measurement design applied to the electronic device.

According to an embodiment, the light concentration change member 213 may perform a role of changing the angle of light emitted by the light-emitting unit 211 to a second direction (or vertical direction) (for example, z-axis) substantially perpendicular to a first direction (e.g., substrate surface direction defined by x,y-axes) and/or a role of concentrating light emitted in the second direction (for example, z-axis) at one point.

According to an embodiment, the light concentration change member 213 may be designed such that light transferred onto the substrate surface in a first direction is changed to a light transferred in a second direction having a predetermined radiation angle.

According to an embodiment, the light concentration change member 213 may include a grating coupler, but is not limited thereto. According to an embodiment, the light concentration change member 213 may be implemented in various structures (for example, a curved micro mirror, a graded refractive index (GRIN) lens, a diffractive optical lens (DOL), a micro lens array (MLA), a diffractive optical lens (DOL), and/or a computer-generated hologram (CGH)) designed to radiate light at a radiation angle in a second direction (or vertical direction) (for example, z-axis) by using a grating coupler, a lens, a slit, or a mirror, or formed by combining some of them.

For example, if the light source is configured to emit light in the first direction (e.g., substrate surface direction defined by x,y-axes) of the substrate surface, the light concentration change member 213 may be a combination of a grating coupler and/or a lens. If the light source is configured to emit light in the vertical direction, that is, the second direction (for example, z-axis) from the substrate surface, the light concentration change member 213 may be implemented as a lens having a refractive index determined to concentrate light.

According to an embodiment, the light-receiving unit 217 may include a photodiode (PD) configured to sense light. The light-receiving unit 217 may convert the intensity of received light to an electric signal and may output the same to the processor 120. The light-receiving unit 217 may selectively sense wavelengths. For example, when the light-emitting unit 211 emits light through a light source in a first waveband, the light-receiving unit 217 may be designed to sense light in the first waveband and not to sense light in other wavebands. For example, the wavelength selectivity of the light-receiving unit 217 may be supported by an optical filter. The light-receiving unit 217 may sense only light that passed through the optical filter. The optical filter may selectively transmit light according to the wavelength.

According to an embodiment, the processor 120 may control the light-emitting unit 211 so as to change the waveband of light generated thereby, according to the usage of biometric measurement. Accordingly, the waveband of light received by the light-receiving unit 217 may be changed.

According to an embodiment, the processor 120 may acquire biometric information, based on light measured from the light-receiving unit 217.

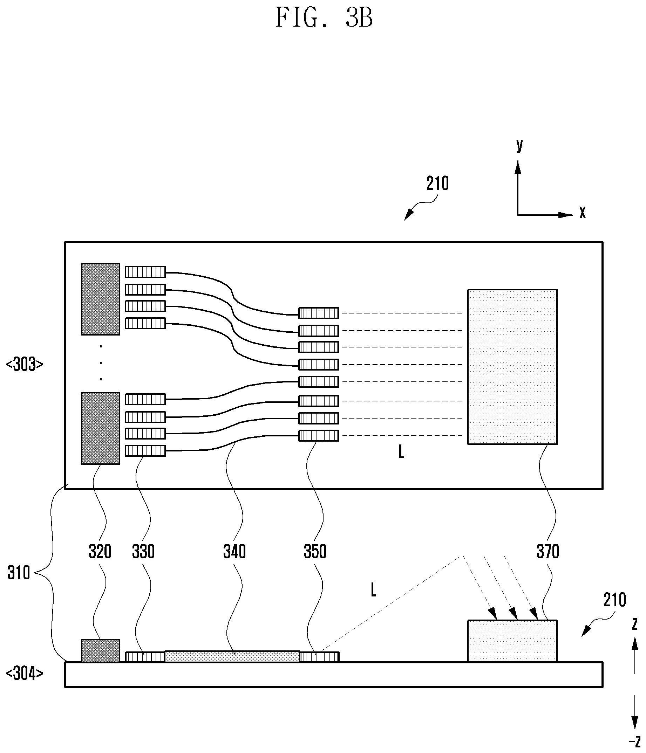

According to an embodiment an optical sensor of an electronic device includes: a light-emitting unit disposed on a substrate, where the light-emitting unit includes light sources configured to emit light in different wavebands; first optical waveguides configured to transfer light emitted through the light-emitting unit in a first direction parallel to a surface of the substrate; and a light concentration change member configured to change an angle of light such that light transferred from the first optical waveguides is discharged in a second direction substantially perpendicular to the first direction by the light concentration change member, where the light concentration change member has a structure configured to concentrate, at one point, light rays transferred from the first optical waveguides in different positions. It will be assumed in the following description that the light concentration change member 213 is an optical sensor implemented by a grating coupler, but this is only an example, and the light concentration change member 213 may be replaced with other structures. FIG. 3A and FIG. 3B illustrate examples of the structure of an optical sensor included in an electronic device according to an embodiment.

Referring to FIG. 3A and FIG. 3B, the biometric sensor or optical sensor (for example, the optical sensor in FIG. 2) 210 included in an electronic device according to an embodiment (for example, the electronic device 101 in FIG. 1) may include a light-emitting unit 320 (for example, the light-emitting unit 211 in FIG. 2) disposed on a substrate 310, a filter (or filter array) 330, an optical waveguide 340, a grating coupler 350 (for example, the light concentration change member 213 in FIG. 2), and a light-receiving unit 370 (for example, the light-receiving unit 217 in FIG. 2). In FIG. 3A and FIG. 3B, <301> and <303> refer to plan views of the optical sensor 210, and <302> and <304> refer to side views of the optical sensor 210. As used herein, the "plan view" is a view in a thickness direction (the second direction, for example, z-axis) of the optical sensor 210, and the "side view" is a view in a direction (e.g., y-axis direction) substantially perpendicular to the thickness direction. The optical sensor 210 is illustrated in FIG. 3A and FIG. 3B in a structure in which eight filters 330 and eight grating couplers 350 are arranged, but this is only an example.

According to an embodiment, the substrate 310 may include a flat plate-type silicon substrate, but is not limited thereto. The substrate 310 may include a printed circuit board (PCB) substrate or a photonic integrated circuit (PIC) substrate.

The light-emitting unit 320, the filter 330, the optical waveguide 340, the grating coupler 350, and the light-receiving unit 370 may be arranged on the substrate 310 so as to be spaced apart from each other by a predetermined distance.

The light-emitting unit (or light-emitting array) 320 may be designed to emit light in different wavebands. The light-emitting unit 320 may include one or more light sources. The light source may include a laser diode (LD) configured to generate light, a light-emitting diode (LED), and/or other light sources.

According to an embodiment, if the light source is implemented by an LD, a filter 330 may be included therein, but if the light source is implemented by an LED, the filter 330 may be omitted.

The filter (or filter array) 330 may selectively filter light emitted by the light-emitting unit 320. For various biometric measurements, the filter 330 may be designed to selectively transmit necessary light only, according to biometric measurement information. For example, in the case of SpO2 measurement, the filters 330 may be designed to transmit an approximately 640 nm waveband and an approximately 940 nm waveband only. In the case of heart rate measurement, a filter 330 may be designed to transmit an approximately 520 nm waveband only. The filter 330 may be variously designed according to biometric measurement information (for example, saturation of percutaneous oxygen (SpO2), heart rate (HR), photoplethysmography (PPG), galvanic skin response (GSR), electrocardiography (ESG), bioelectrical impedance, blood glucose, hemoglobin concentration, neutral fat, body composition, alcohol index) supported by the electronic device 101.

The optical waveguide 340 may refer to an optical channel for transmitting light. The optical waveguide 340 may transfer light emitted by the light-emitting unit 320 or light that has passed through the filter 330 to the grating coupler 350 in the first direction (e.g., substrate surface direction defined by x,y-axes) of the substrate surface. The optical waveguide 340 may extend along one direction parallel to the upper surface of the grating coupler 350.

The grating coupler 350 may change the angle of light transferred from the optical waveguide 340 toward a second direction (for example, z-axis) substantially perpendicular to the first direction of the surface of the substrate 310 and then radiate the light. The grating coupler 350 may be designed to change the path of light L to a predetermined radiation angle with respect to the surface of the substrate 310, as illustrated at <302> and <304>. The radiation angle of the grating coupler 350 may vary depending on design.

Light L radiated in the second direction through the grating coupler 350 may reach a light irradiation region 360 spaced apart from the substrate 310 by a predetermined distance. The light irradiation region 360 may correspond to a region capable of contacting a biometric measurement subject (for example, the user's body), for example.

Although not illustrated in the drawings, the optical sensor 210 may have a light-receiving unit 370 positioned to be able to receive light L reflected from the biometric measurement subject (for example, the user's body). The light-receiving unit 370 may convert the intensity of received light L to an electric signal and output the same to the processor 120.

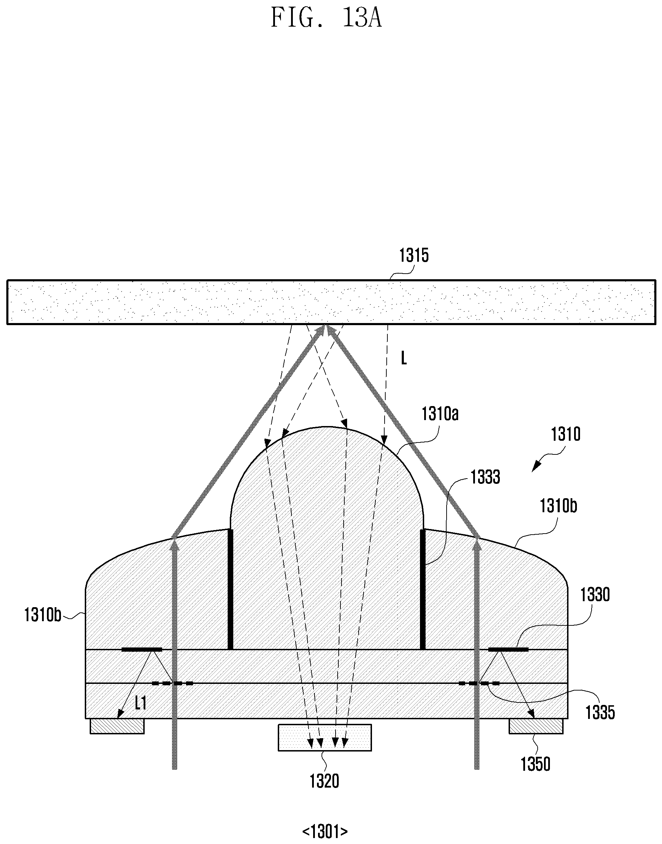

Although not illustrated in the drawings, according to an embodiment, the optical sensor 210 in FIG. 3A and FIG. 3B may be disposed on the electronic device 101 together with one of lens structures illustrated in FIG. 13A to FIG. 13C.

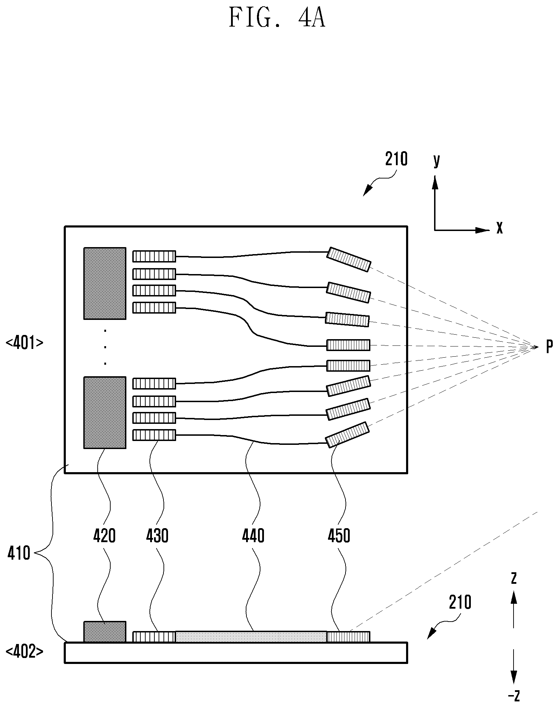

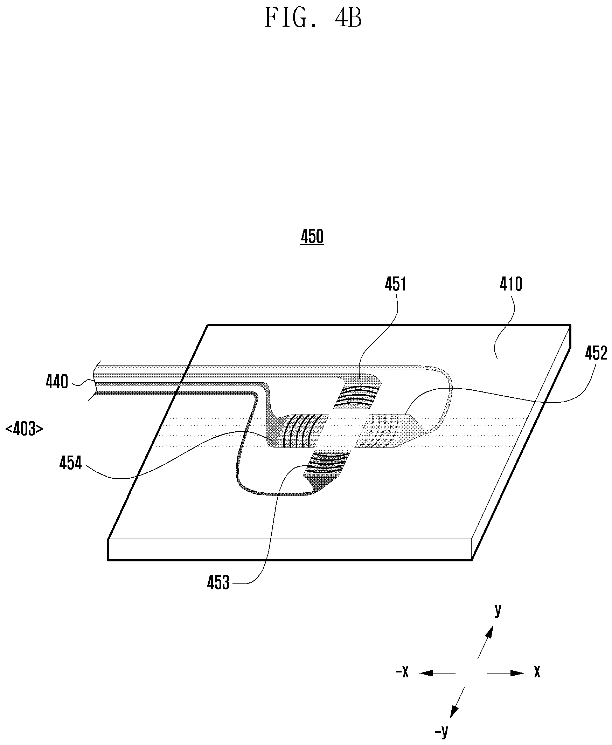

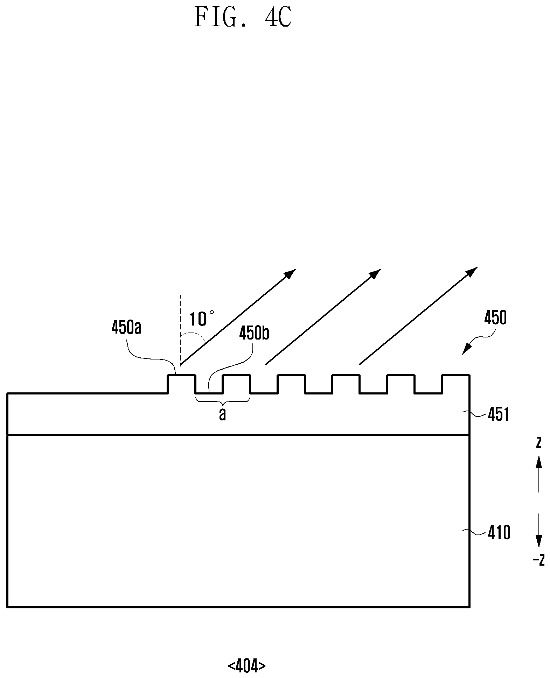

FIG. 4A illustrates an example of the structure of an optical sensor included in an electronic device according to an embodiment. FIG. 4B and FIG. 4C illustrate the structure of a grating coupler according to an embodiment. FIG. 4B is a perspective view of the grating couplers 450, and FIG. 4C is a cross-sectional view of the grating couplers 450.

Referring to FIG. 4A, the biometric sensor or optical sensor 210 included in an electronic device according to an embodiment (for example, the electronic device 101 in FIG. 1) may include a light-emitting unit 420 (for example, the light-emitting unit 211 in FIG. 2) disposed on a substrate 410, a filter (or filter array) 430 configured to selectively filter emitted by the light-emitting unit 420, an optical waveguide 440 configured to transfer light that has passed through the filter 430, and a grating coupler 450 (for example, the light concentration change member 213 in FIG. 2) configured to change the angle of light transferred from the optical waveguide 440 toward the z-axis direction (i.e., thickness direction) of the substrate 410.

Although not illustrated in the drawings, a light-receiving unit (not illustrated) may be disposed on the substrate 410. In FIG. 4A, <401> refers to a plan view of the optical sensor 210, and <402> refers to a side view of the optical sensor 210.

In the example in FIG. 4A, grating couplers 450 may be designed in a structure such that light transferred from the optical waveguide 440 in the first direction (e.g., substrate surface direction defined by x,y-axes) is radiated in a second direction having a predetermined radiation angle with regard to the first direction, and rays of light L are concentrated at one point P. The grating couplers 350 illustrated in FIG. 3A are disposed on the substrate 310 in parallel to each other, and it may be identified that light rays are radiated from respective grating couplers 350 in a parallel manner. On the other hands, the grating couplers 450 illustrated in FIG. 4A may be arranged on the substrate 410 so as to deviate from each other by a predetermined angle such that radiated light rays are concentrated at one point P. Hereinafter, the light-emitting unit 420, the filter 430, the optical waveguide 440, and the light-receiving unit (not illustrated) have the same configurations as in FIG. 3, and detailed descriptions thereof will thus be omitted.

According to an embodiment, the grating couplers 450 may be designed in various structures such that light rays from different light sources or in different wavebands are concentrated at one point. For example, the grating couplers 450 may be designed in the structure as in FIG. 4B. As illustrated in FIG. 4B, the first grating coupler 451 may be designed to have a predetermined radiation angle with respect to the x-axis direction, the second grating coupler 452 may be designed to have a predetermined radiation angle with respect to the y-axis direction, the third grating coupler 453 may be designed to have a predetermined radiation angle with respect to the -x-axis direction, and the fourth grating coupler 454 may be designed to have a predetermined radiation angle with respect to the -y-axis direction. Light rays radiated from the four directions may be concentrated at one point (e.g., center point).

According to an embodiment, the grating coupler 450 may be designed to have different grating pattern periods according to the light propagation direction. For example, the grating coupler 450 may have a grating pattern formed by the first grating coupler 451 (e.g., a silicon layer) disposed on the substrate 410, as illustrated in FIG. 4C. The upper end of the first grating coupler 451 (e.g., a silicon layer) may have a grating pattern formed to include a protrusion 450a and a trench 450b (or a pattern in which a trench and a protrusion each are repeated at a predetermined period "a"). The first grating coupler 451 (e.g., a silicon layer) may be connected to the optical waveguide 440. Light transferred from the optical waveguide 440 may be reflected, scattered, and diffracted by the trenches, thereby changing the light radiation angle. The period a of the grating pattern may be determined by Equation 1 below:

$$[\text{Equation 1}]$$

$$\sin(\theta) = \frac{n_{\text{eff}} - \dfrac{\lambda}{a}}{n_{\text{air}}}$$

Equation 1 above is only an example to aid understanding, and is not limited thereto, and may be modified, applied, or expanded in various ways.

The grating couplers 450 may be designed to have different periods with regard to respective light wavebands. For example, in the case of the optical sensor 210 illustrated in FIG. 4A, four filters 430 and four grating couplers 450 disposed in the first region may be designed to have different wavebands and different periods a as in Table 1, and four filters 430 and four grating couplers 450 disposed in the second region may be designed to have different wavebands and different periods a as in Table 2, but this is only an example.

[Table 1]

| Wavelength ($\mu$m) | 2.00 | 2.01 | 2.02 | 2.03 |
|---|---|---|---|---|
| a (nm) | 860 | 864 | 868 | 873 |

[Table 2]

| Wavelength ($\mu$m) | 2.41 | 2.41 | 2.42 | 2.43 |
|---|---|---|---|---|
| a (nm) | 1036 | 1036 | 1040 | 1045 |

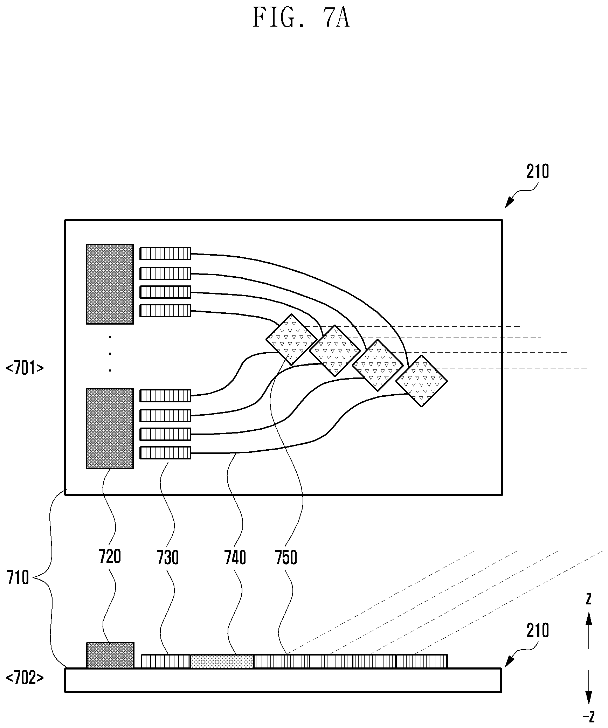

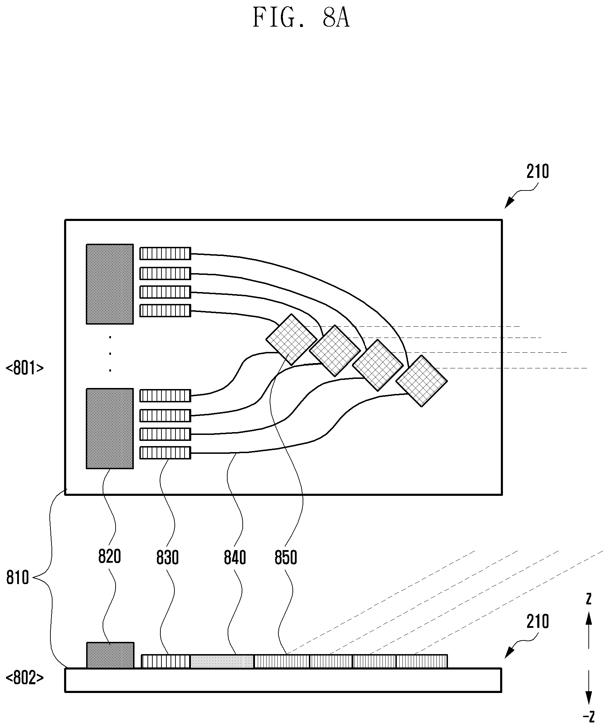

The grating pattern in FIG. 4C refers to a structure in which light is radiated toward the Z-axis direction with regard to only one wavelength, but the grating couplers 450 may be designed, as illustrated in FIG. 7A and FIG. 8A, to have grating patterns formed differently according to the light propagation direction such that the light radiation angle is changed with regard to two or more wavelengths as well.

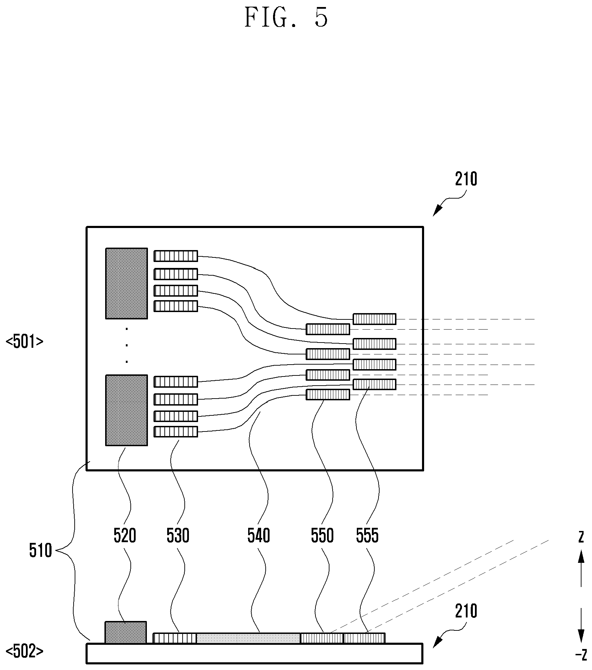

FIG. 5 illustrates an example of the structure of an optical sensor included in an electronic device according to an embodiment.

Referring to FIG. 5, the biometric sensor or optical sensor 210 included in an electronic device according to an embodiment (for example, the electronic device 101 in FIG. 1) may be designed in various disposition configurations in order to minimize the disposition region. In FIG. 5, <501> refers to a plan view of the optical sensor 210, and <502> refers to a side view of the optical sensor 210.

As an example, the optical sensor 210 includes a light-emitting unit 520 (for example, the light-emitting unit 211 in FIG. 2) disposed on a substrate 510, a filter (or filter array) 530 configured to selectively filter emitted by the light-emitting unit 520, and an optical waveguide 540 configured to transfer light that has passed through the filter 530, and may have a disposition structure of grating couplers 550 and 555 spaced apart by a predetermined distance in a parallel matrix and in which the rear end of the first grating coupler 550 (for example, the light concentration change member 213 in FIG. 2) and the front end of the second grating coupler 555 (for example, the light concentration change member 213 in FIG. 2) intersect in parallel. The first grating coupler 550 may be interposed between two second grating couplers 555 in a view in the first direction. In this case, the optical sensor 210 may further include a lens structure (not illustrated) (for example, the lens structure in FIG. 13A to FIG. 13C) for concentrating light rays emitted from the first grating coupler 550 and the second grating coupler 555 in different positions to one point.

In the example in FIG. 5, the first grating coupler 550 and the second grating coupler 555 are arranged in parallel to each other in the same region, but may also be arranged so as to deviate from each other by a predetermined angle such that light rays radiated from the first grating coupler 550 and the second grating coupler 555 are concentrated at one point, as illustrated in FIG. 4A, while maintaining the arrangement configuration in FIG. 5.

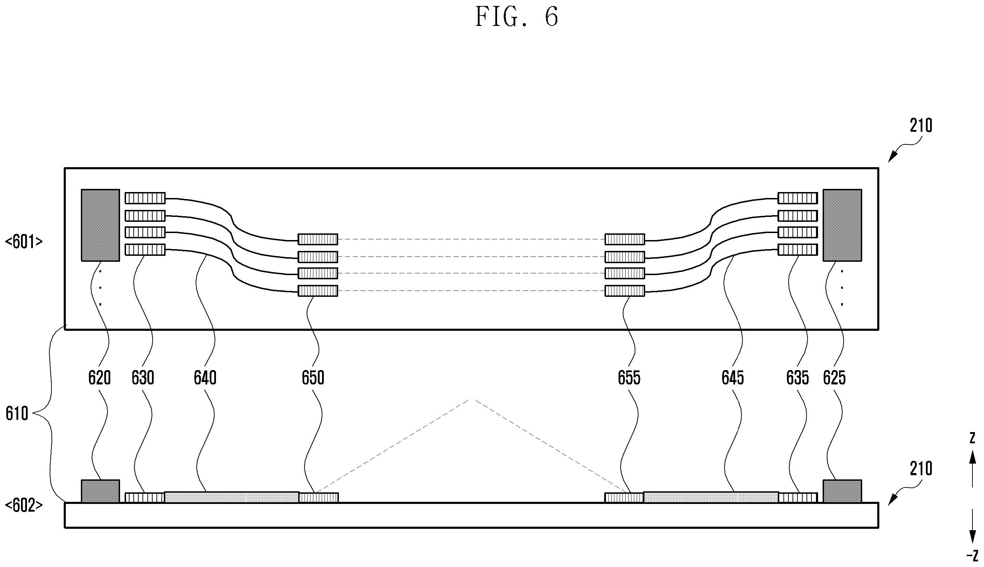

FIG. 6 illustrates an example of the structure of an optical sensor included in an electronic device according to an embodiment.

Referring to FIG. 6, the biometric sensor or optical sensor 210 included in an electronic device according to an embodiment (for example, the electronic device 101 in FIG. 1) may have components of the optical sensor disposed so as to correspond to the length of the substrate. In FIG. 6, <601> refers to a plan view of the optical sensor 210, and <602> refers to a side view of the optical sensor 210. As an example, the optical sensor 210 may have serial arrangement instead of the parallel arrangement in FIG. 3A/FIG. 4A/FIG. 5. The optical sensor 210 may have a first light-emitting unit 620, a first filter 630, a first optical waveguide 640, and a first grating coupler 650 disposed in the first region of the substrate 610, and may have a second light-emitting unit 625, a second filter 635, a second optical waveguide 645, and a second grating coupler 655 disposed in the second region of the substrate 610.

Although not illustrated in the drawings, light-receiving units (not illustrated) may be disposed to the position in which light rays radiated from the first grating coupler 650 and the second grating coupler 655 are collected (e.g., center position in FIG. 6).

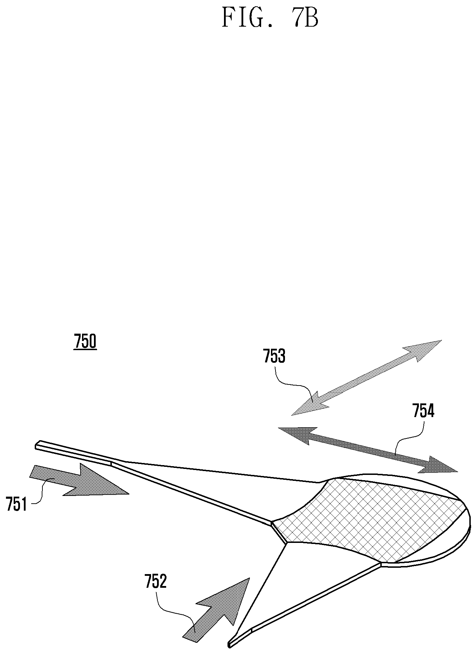

FIG. 7A and FIG. 7B illustrate examples of the structure of an optical sensor included in an electronic device according to an embodiment.

Referring to FIG. 7A and FIG. 7B, the biometric sensor or optical sensor 210 included in an electronic device according to an embodiment (for example, the electronic device 101 in FIG. 1) may have grating couplers 750 designed in a lattice structure such that the radiation angle of light incident from one or more paths can be changed and polarized in different directions depending on the paths of the incident light. In FIG. 7A, <701> refers to a plan view of the optical sensor 210, and <702> refers to a side view of the optical sensor 210. The optical sensor 210 may have light-emitting units 720, filters 730, optical waveguides 740, and grating couplers 750 disposed on the substrate 710 such that the grating couplers 750 are designed in a grating configuration as in the structure in FIG. 7B, thereby reducing the number of the grating couplers 750. As illustrated in FIG. 7B, the light ray 751 coming through the first path may have the first diffraction (or polarized) direction 753, and the light ray 752 coming through the second path may have the second diffraction (or polarized) direction 754. Therefore, the light-receiving units receiving the polarized light ray 751 and light ray 752 may distinguish them from each other, and changing the light radiation angle with regard to two different light rays through one grating coupler 750 is possible.

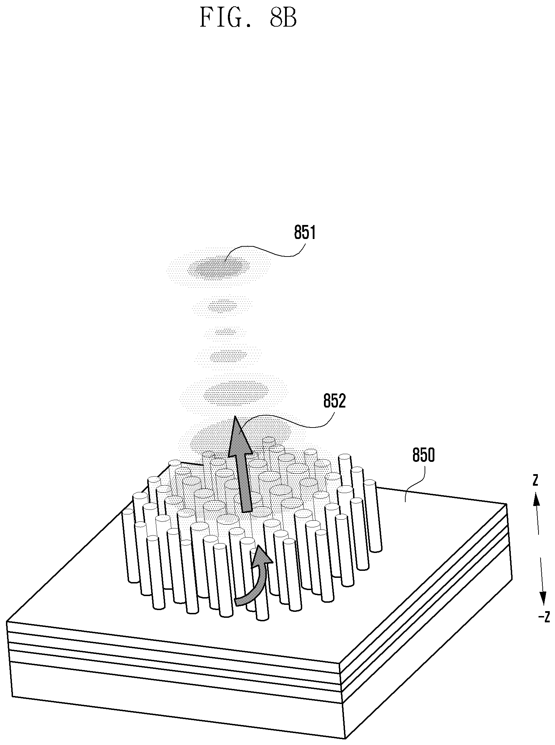

FIG. 8A and FIG. 8B illustrate examples of the structure of an optical sensor included in an electronic device according to an embodiment.

Referring to FIG. 8A and FIG. 8B, the biometric sensor or optical sensor 210 included in an electronic device according to an embodiment (for example, the electronic device 101 in FIG. 1) may have grating couplers 750 designed such that rod-shaped structures are disposed at a predetermined period.

In FIG. 8A, <801> refers to a plan view of the optical sensor 210, and <802> refers to a side view of the optical sensor 210. The optical sensor 210 may have light-emitting units 820, filters 830, optical waveguides 840, and grating couplers 850 disposed on the substrate 810 such that the grating couplers 850 are designed in rod shapes as in the structure in FIG. 8B, thereby reducing the number of grating couplers 850.

It may be identified that, compared with examples in FIG. 3A/FIG. 4/FIG. 5, the number of grating couplers 750 and 850 in the examples in FIG. 7A and FIG. 8A is reduced from 8 to 4, thereby making the optical sensor 210 compact.

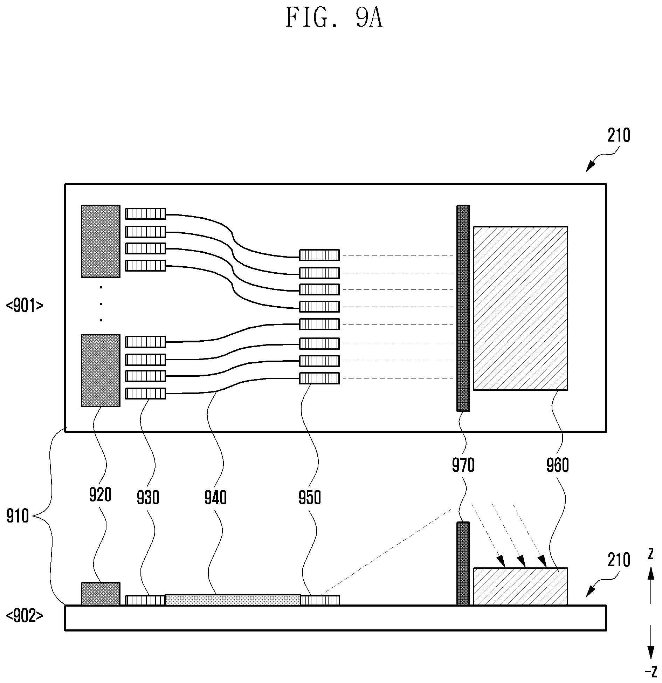

FIG. 9A to FIG. 9C illustrate examples of the structure of an optical sensor included in an electronic device according to an embodiment.

Referring to FIG. 9A to FIG. 9C, the biometric sensor or optical sensor 210 included in an electronic device according to an embodiment (for example, the electronic device 101 in FIG. 1) may include blocking barriers (for example, 970, 973, and 975) configured to block interference between emitted light and received light. The blocking barriers 970, 973, and 975 may be configured to block light emitted by light-emitting units 920 so as not to leak to light-receiving units 960, thereby improving the signal-to-noise ratio (NSR) of light. The blocking barriers 970, 973, and 975 may be made of a light-blocking material that does not transmit light. In FIGS. 9A to 9C, <901>, <903>, and <905> refer to plan views of the optical sensor 210, and <902>, <904>, and <906> refer to side views of the optical sensor 210.

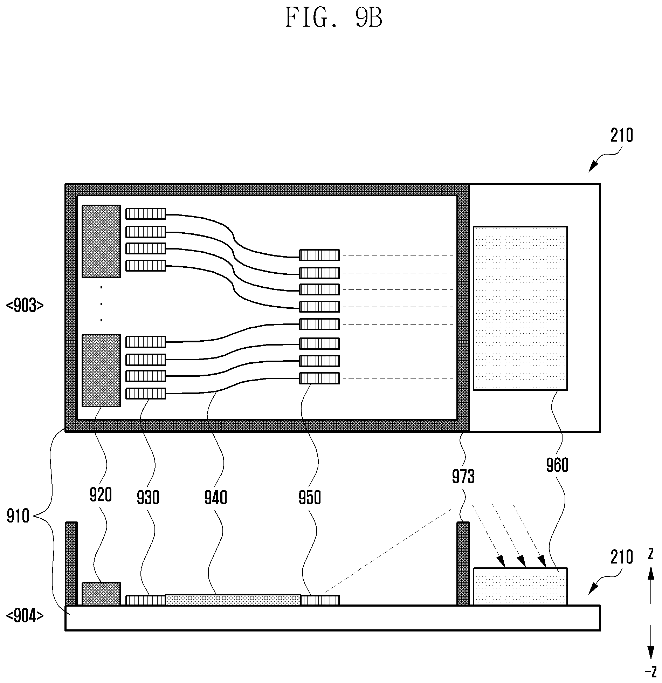

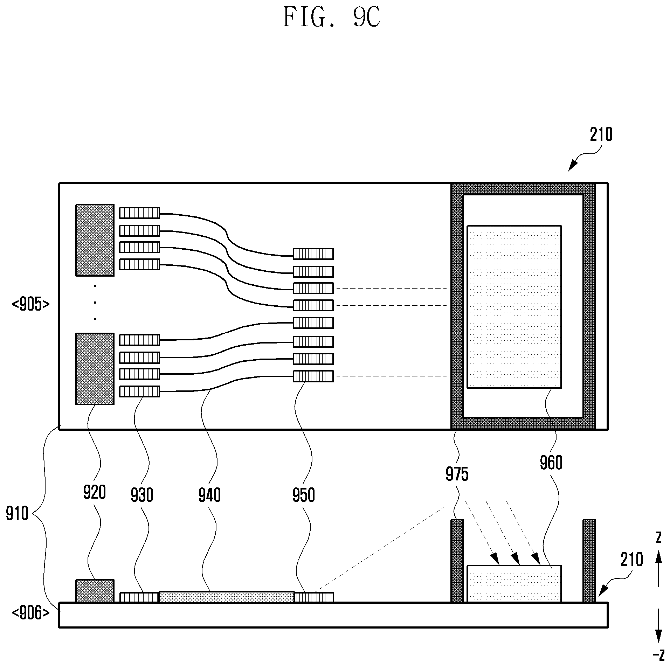

As an example, the optical sensor 210 may have light-emitting units 920, filters 930, optical waveguides 940, grating couplers 950, and light-receiving units 960 disposed on the substrate 910. According to an embodiment, the blocking barriers (for example, 970, 973, and 975) may be disposed in various configurations. For example, as illustrated in FIG. 9A, the block barrier 970 may be implemented in a bar shape between the grating couplers 950 and the light-receiving unit 960. As illustrated in FIG. 9B, the block barrier 973 may be implemented in an edge shape surrounding the outer peripheral regions of the light-emitting units 920, the filters 930, the optical waveguides 940, and the grating couplers 950, or as illustrated in FIG. 9C, the block barrier 975 may be implemented in an edge shape surrounding the outer peripheral region of the light-receiving unit 960.

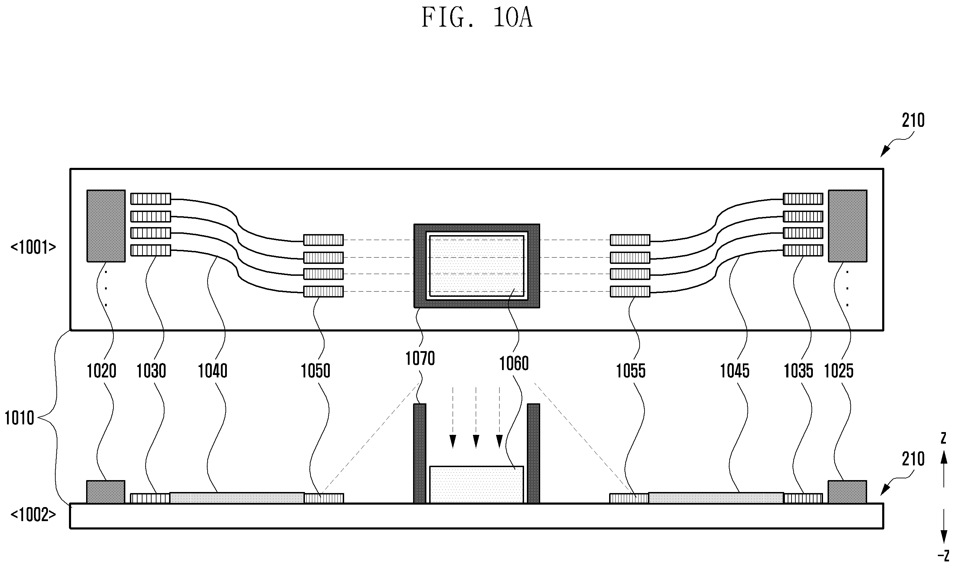

FIG. 10A to FIG. 10C illustrate examples of the structure of an optical sensor included in an electronic device according to an embodiment.

Referring to FIG. 10A to FIG. 10C, the substrate 1010 of the biometric sensor or optical sensor 210 included in an electronic device according to an embodiment (for example, the electronic device 101 in FIG. 1) may be implemented in various shapes. In FIG. 10A, <1001> refers to a plan view of the optical sensor 210, and <1002> refers to a side view of the optical sensor 210.

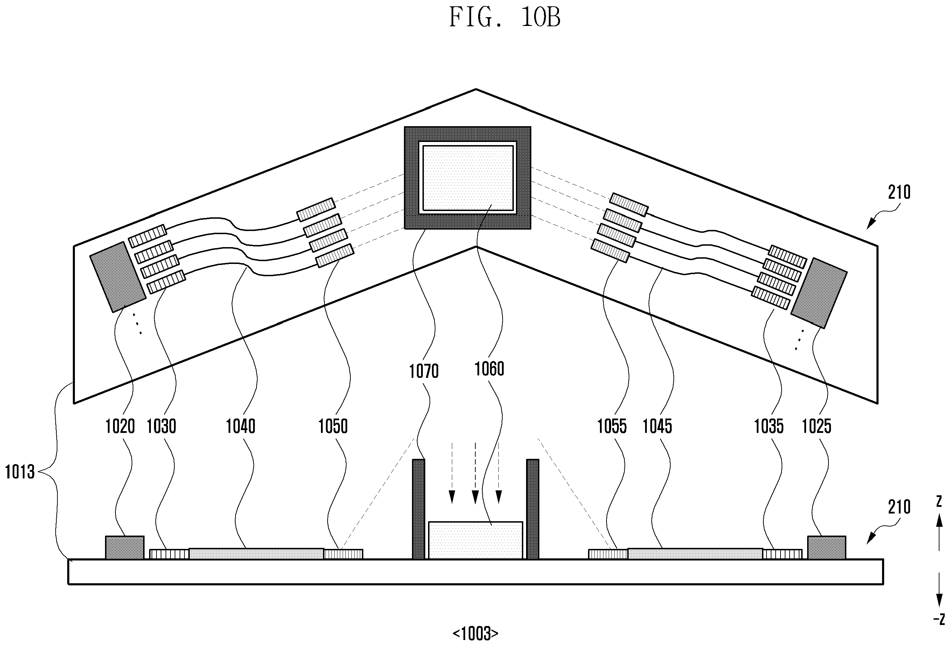

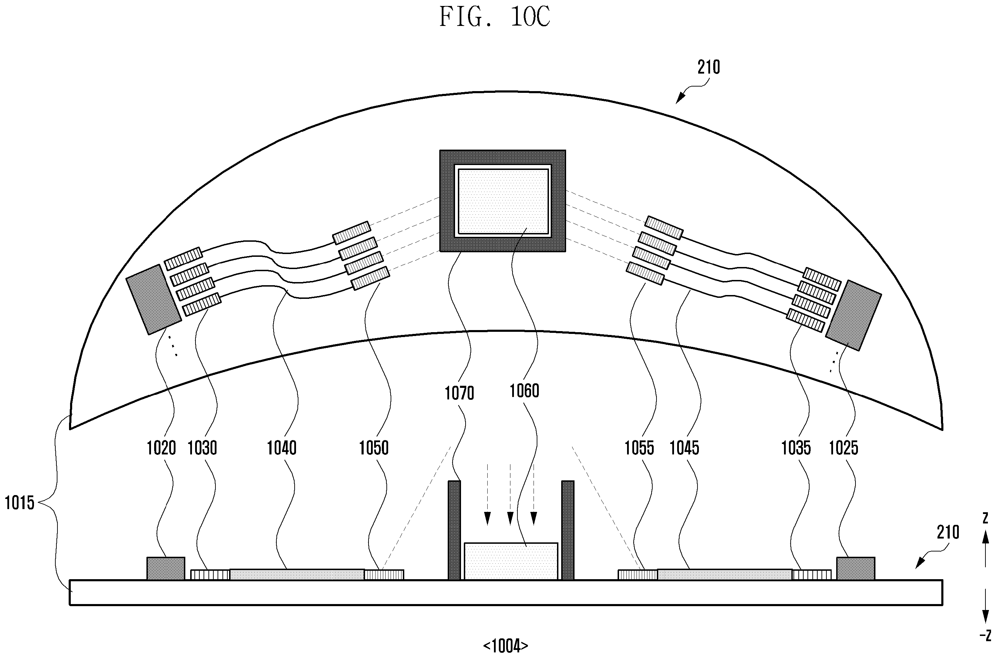

As an example, similarly to the serial arrangement structure illustrated in FIG. 6, the optical sensor 210 may have a first light-emitting unit 1020, a first filter 1030, a first optical waveguide 1040, and a first grating coupler 1050 disposed in the first region of the substrate 1010, and may have a second light-emitting unit 1025, a second filter 1035, a second optical waveguide 1045, and a second grating coupler 1055 disposed in second first region of the substrate 1010. A light-receiving unit 1060 may be disposed so as to correspond to the position in which light rays radiated from the first grating coupler 1050 and the second grating coupler 1055 are concentrated, and a blocking barrier 1070 may be disposed as an edge surrounding the outer periphery of the light-receiving unit 1060. As an example, the substrate 1010 may be implemented as a rectangular substrate 1010 as illustrated at <1001> in FIG. 10A, as a substrate 1013 having the shape illustrated at <1003> in FIG. 10B, or as a substrate 1015 having the shape illustrated at <1004> in FIG. 10C, and various other shapes may be applied to the substrate.

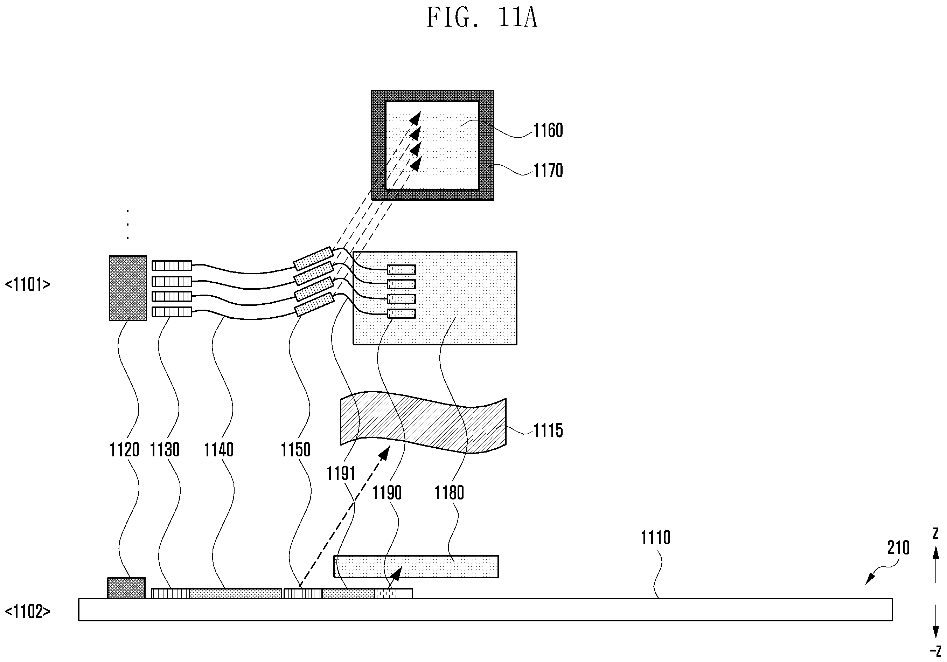

FIG. 11A to FIG. 11C illustrate examples of the structure of an optical sensor included in an electronic device according to an embodiment.

Referring to FIG. 11A to FIG. 11C, the biometric sensor or optical sensor 210 included in an electronic device according to an embodiment (for example, the electronic device 101 in FIG. 1) may include a monitoring unit 1180 configured to monitor light radiated or output from the grating coupler 1150. In FIGS. 11A to 11C, <1101>, <1103>, and <1105> refer to plan views of the optical sensor 210, and <1102>, <1104>, and <1106> refer to side views of the optical sensor 210. As an example, the optical sensor 210 may have a light-emitting unit 1120, a filter 1130, an optical waveguide 1140, a grating coupler 1150, and a light-receiving unit 1160 disposed on the substrate 1110. A blocking barrier 1170 may be disposed as an edge surrounding the outer periphery of the light-receiving unit 1160.

In the case of the grating coupler 1150, light transferred on the surface of the substrate 1110 is radiated after an angle of the light changes to the second direction to transmit to the biometric measurement subject (for example, the user's body 1115), and there may be some light rays remaining on the surface of the substrate 1110.

According to an embodiment, the monitoring unit 1180 may have a sub-waveguide 1191 and a sub-grating coupler 1190 disposed to be connected to the output end of the grating coupler 1150 in order to monitor the remaining light of the grating coupler 1150, as illustrated in FIG. 11A. The sub-grating coupler 1190 and the sub-waveguide 1191 may be disposed for each grating coupler 1150 such that the intensity and wavelength of light radiated from each grating coupler 1150 can all be monitored.

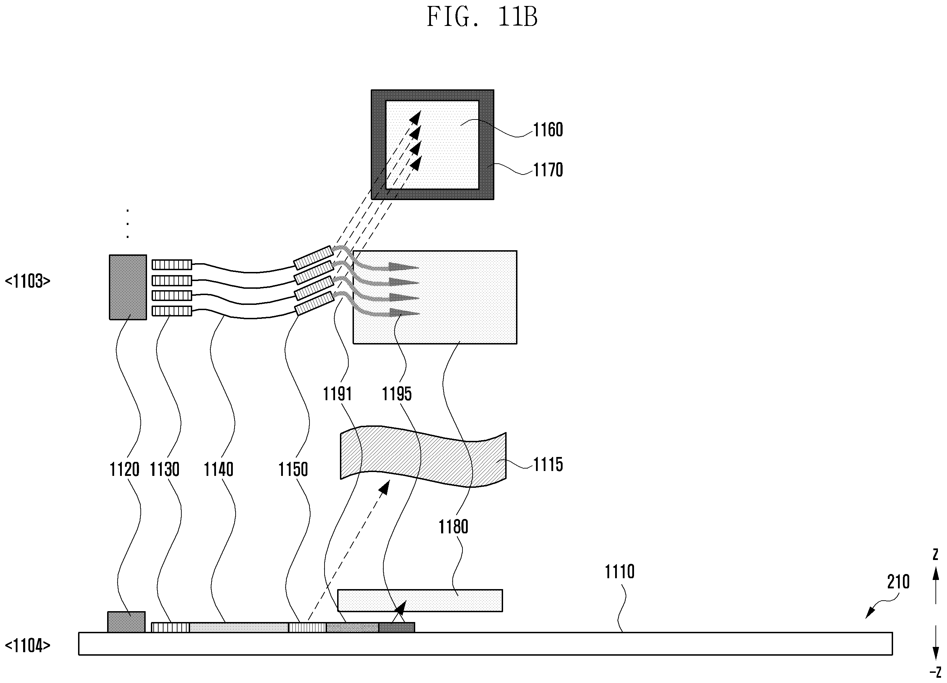

According to an embodiment, the monitoring unit 1180 may have a sub-waveguide 1191, and the end 1195 of the sub-waveguide 1191 is implemented as a mirror having a tapered shape or having a specific angle, in order to monitor the remaining light of the grating coupler 1150, as illustrated in FIG. 11B. The tapered shape of the end 1195 of the sub-waveguide 1191 ensures that remaining light of the grating coupler 1150 is spread wide and then incident onto the monitoring unit 1180.

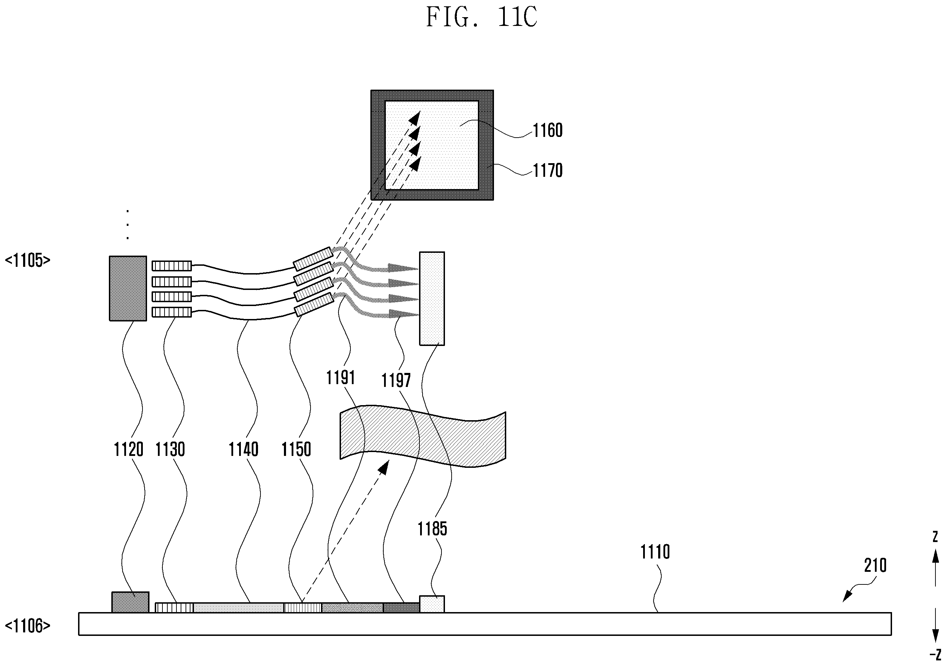

According to an embodiment, the monitoring unit 1185 may have a sub-waveguide 1191, and the end 1197 of the sub-waveguide 1191 is implemented in an edge-illuminated type while having a taped shape, as illustrated in FIG. 11C.

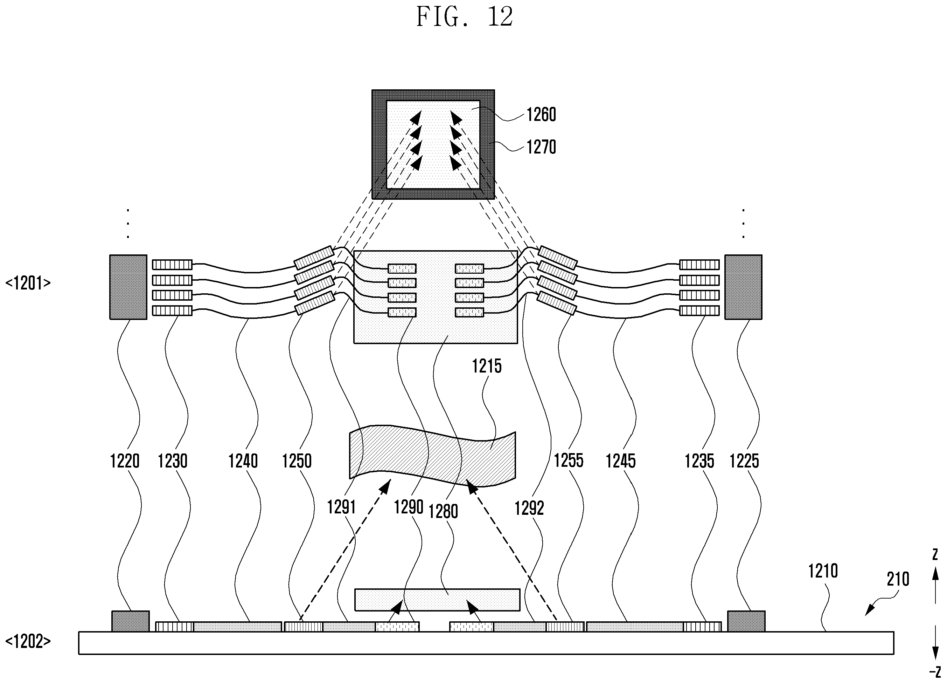

FIG. 12 illustrates an example of the structure of an optical sensor included in an electronic device according to an embodiment.

Referring to FIG. 12, the substrate 1010 of the biometric sensor or optical sensor 210 included in an electronic device according to an embodiment (for example, the electronic device 101 in FIG. 1) may have, similarly to the serial arrangement structure illustrated in FIG. 6, a first light-emitting unit 1220, a first filter 1230, a first optical waveguide 1240, and a first grating coupler 1250 disposed in the first region of the substrate 1210, and may have a second light-emitting unit 1225, a second filter 1235, a second optical waveguide 1245, and a second grating coupler 1255 disposed in second first region of the substrate 1210. In FIG. 12, <1201> refers to a plan view of the optical sensor 210, and <1202> refers to a side view of the optical sensor 210. In this case, a monitoring unit 1280 may be disposed between the first grating coupler 1250 in the first region and the second grating coupler 1255 in the second region. The monitoring unit 1280 may monitor all light radiated through each grating coupler 1250 through a sub-grating coupler 1290 connected to each grating coupler 1250. The optical sensor 210 may have a light-receiving unit 1260 disposed to the position in which light rays radiated from the first grating coupler 1250 and the second grating coupler 1255 are collected, and may have a blocking barrier 1270 disposed as an edge surrounding the outer periphery of the light-receiving unit 1260. Here, 1215 indicates the biometric measurement subject (for example, the user's body).

FIG. 13A to FIG. 13C illustrate examples of a lens structure included in an electronic device according to an embodiment.

Referring to FIG. 13A to FIG. 13C, the biometric sensor or optical sensor 210 included in an electronic device according to an embodiment (for example, the electronic device 101 in FIG. 1) may include a lens structure 1310 for concentrating light rays radiated through grating couplers (for example, the grating coupler 350 in FIG. 3A, the grating coupler 550 in FIG. 5, the first grating coupler 650 and the second grating coupler 655 in FIG. 6, the grating coupler 750 in FIG. 7A, the grating coupler 850 in FIG. 8A, the grating coupler 950 in FIG. 9A to FIG. 9C, the first grating coupler 1050 and the second grating coupler 1055 in FIG. 10A to FIG. 10C, the grating coupler 1150 in FIG. 11A to FIG. 11C, the first grating coupler 1250 and the second grating coupler 1255 in FIG. 12) in various positions at one point. Although not illustrated in the drawings, the light-emitting unit, the filter, the optical waveguide, and the grating coupler of the optical sensor 210 may be omitted in FIG. 13A to FIG. 13C.

The lens structure 1310 may be disposed as a separate component from the optical sensor 210.

The lens structure 1310 may be designed to have multiple curvatures and to be able to concentrate light through the lens and to monitor the output. As an example, as illustrated in FIG. 13A, the lens structure 1310 may include a first region having a first curvature 1310a at the center, and a second region having a second curvature 1310b on the outer periphery. Light emitted through the second curvature 1310b may be concentrated at the biometric measurement subject (for example, the user's body 1315), and light L reflected or scattered at the subject may be concentrated at the light-receiving unit 1320 through the first region having the first curvature 1310a. The lens structure 1310 may have a blocking wall 1333 disposed between the first region and the second region to block emitted light and collected light L from each other.

The lens structure 1310 may be designed such that a part of light emitted from the light-emitting unit or the grating coupler is scattered toward the subject by using a diffractive optical element (DOE) 1335, and the scattered light L1 is reflected downward at the overlying reflecting surface 1330 and then monitored through the monitoring unit 1350.

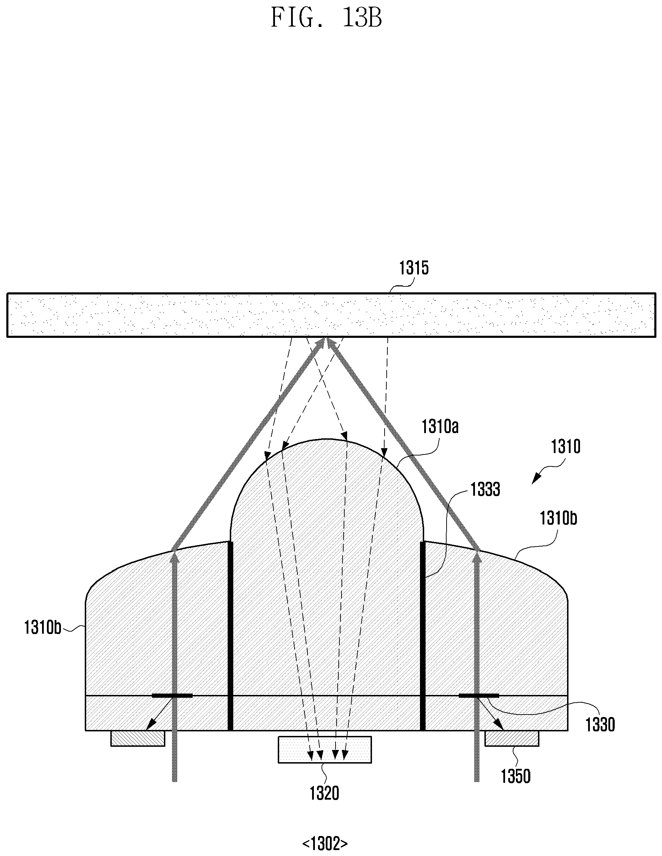

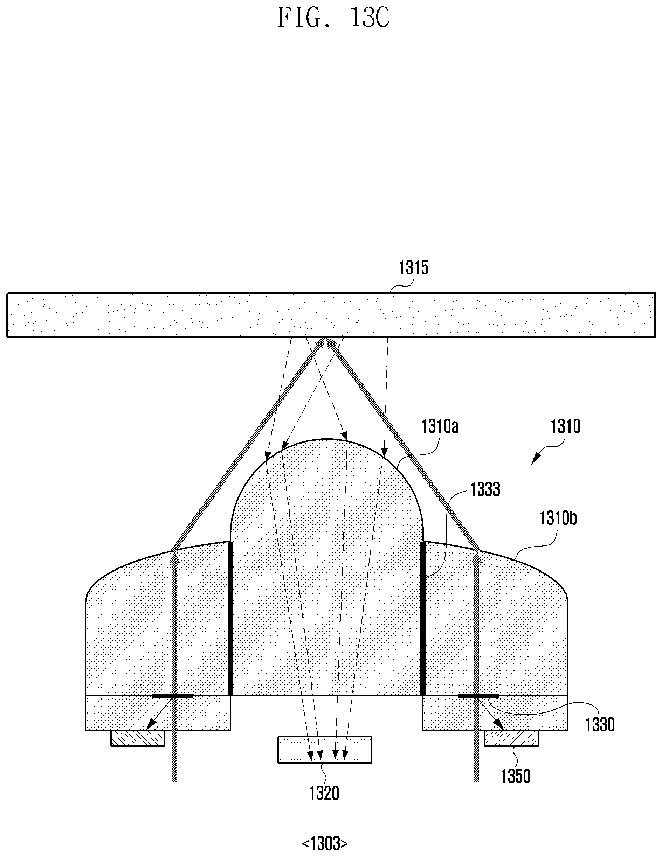

According to an embodiment, the lens structure 1310 may be designed to have a reflecting surface 1330 without a diffractive optical element (DOE) 1335 as in the structure in FIG. 13B or FIG. 13C. This is only an example, and the lens structure may also be designed in other structures.

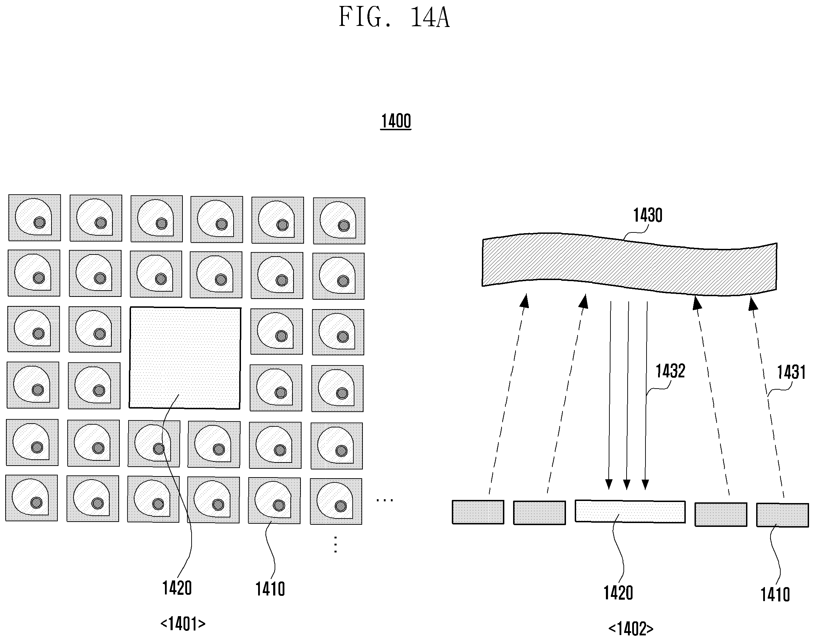

FIG. 14A to FIG. 14C illustrate examples of a lens structure included in an optical sensor according to an embodiment.

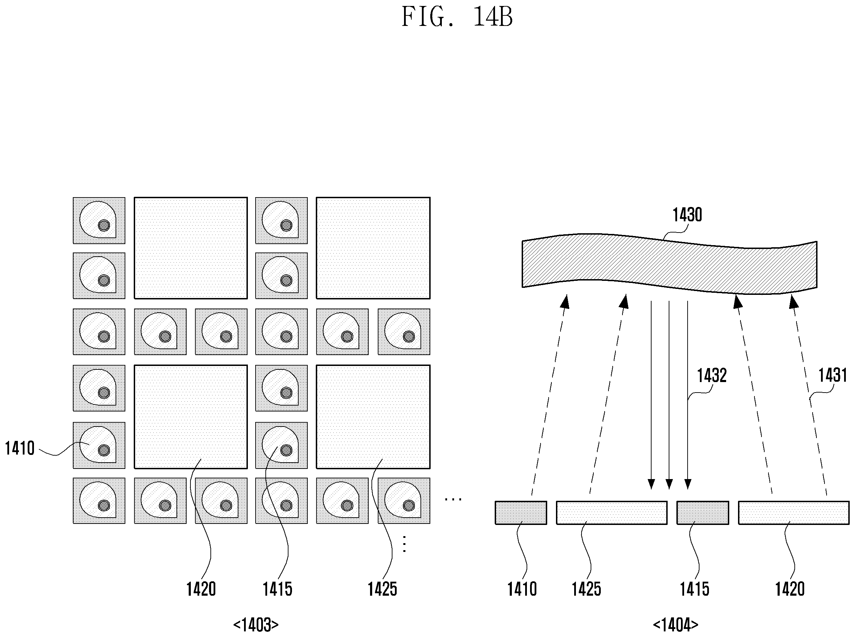

Referring to FIG. 14A to FIG. 14C, according to an embodiment, the biometric sensor or optical sensor (for example, the optical sensor 210 in FIG. 2) 1400 included in an electronic device (for example, the electronic device 101 in FIG. 1) may include a combination of a light-emitting unit (for example, the light-emitting unit in FIG. 2) of a light source 1410 and 1415 (for example, a vertical cavity surface emitting laser (VCSEL)) configured to radiate light 1431 vertically and a lens structure 1450, 1440. In the examples in FIG. 14A to FIG. 14C, grating couplers may be omitted. In FIGS. 14A and 14B, <1401> and <1403> refer to plan views of the optical sensor 210, and <1402> and <1404> refer to side views of the optical sensor 210.

As an example, a light-emitting unit including multiple VCSEL light sources 1410 and 1415 may be arranged to surround one light-receiving unit 1420 as illustrated in FIG. 14A, or multiple light-receiving units 1420 and 1425 may be disposed as illustrated in FIG. 14B. Depending on the case, a blocking barrier (not illustrated) may be disposed between the light sources 1410 and 1415 and the light-receiving units 1420 and 1425. Here, 1430 indicates the biometric measurement subject (for example, the user's body), 1432 indicates light ray reflected from the biometric measurement subject 1430.

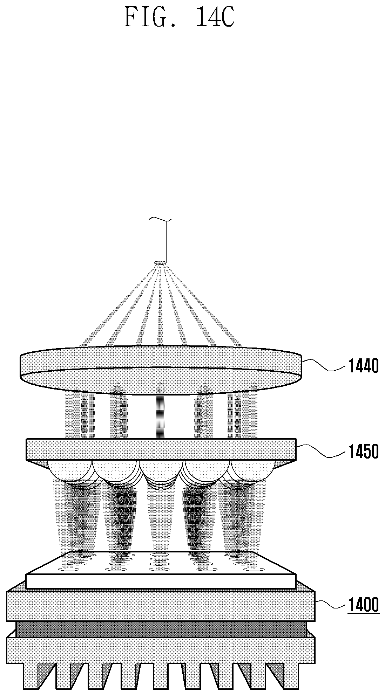

As illustrated in FIG. 14C, the optical sensor 1400 may be combined with a lens structure configured to gather light rays radiated vertically in various positions through the first lens 1450 and to concentrate them at one point through the second lens 1440. For example, the first lens 1450 may include a micro lens array, and the second lens 1440 may include the lens structure illustrated in FIG. 13A to FIG. 13C, but this is only an example, and other lens types may be applicable.

According to an embodiment, an optical sensor includes: a light-emitting unit disposed on a substrate, where the light-emitting unit includes light sources configured to emit light in different wavebands.

According to an embodiment an optical sensor includes first optical waveguides configured to transfer light emitted through the light-emitting unit in a first direction parallel to a surface of the substrate.

According to an embodiment an optical sensor includes a light concentration change member configured to change an angle of light such that light transferred from the first optical waveguides is discharged in a second direction substantially perpendicular to the first direction by the light concentration change member.

According to an embodiment, the light concentration change member may have a structure such that light rays transferred in different positions are concentrated at one point by the light concentration change member.

According to an embodiment, the light sources may include laser diodes or light-emitting diodes.

According to an embodiment, the light-emitting unit may further include filters configured to filter in a specific waveband only, in case that the light sources are laser diodes.

According to an embodiment, the light concentration change member may include multiple grating couplers.

According to an embodiment, the light concentration change member may include a grating coupler, a lens, a slit, or a mirror and may be configured to radiate light at a predetermined radiation angle with respect to the second direction.

According to an embodiment, the multiple grating couplers may be disposed in a lattice structure so as to change a radiation angle of first light transferred through a first path and second light transferred through a second path.

According to an embodiment, the multiple grating couplers may include rod-shaped structures.

According to an embodiment, the multiple grating couplers may be spaced apart by a predetermined distance in a parallel matrix and are arranged such that a longitudinal end of a grating coupler of the multiple grating couplers and a longitudinal front end of another grating coupler of the multiple grating couplers intersect with each other.

According to an embodiment, wherein the substrate may include a printed circuit board (PCB) substrate or a photonic integrated circuit (PIC) substrate.

According to an embodiment an electronic device includes a lens structure configured to concentrate, at the one point, light rays discharged through the multiple grating couplers at a predetermined radiation angle with respect to the second direction.

According to an embodiment an electronic device includes an optical sensor.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

"substantially perpendicular" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, " substantially perpendicular" can mean within one or more standard deviations, or within $\pm$ 10° or 5° of the right perpendicular angle.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the "non-transitory" storage medium is a tangible device, and may not include a signal (e.g., an electromagnetic wave), but this term does not differentiate between where data is semipermanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

**Claims**

1. 1. An optical sensor comprising:

   a light-emitting unit disposed on a substrate, the light-emitting unit comprising light sources configured to emit light in different wavebands;
   first optical waveguides configured to transfer light emitted through the light-emitting unit in a first direction parallel to a surface of the substrate; and
   a light concentration change member configured to change an angle of light such that light transferred from the first optical waveguides is discharged in a second direction substantially perpendicular to the first direction by the light concentration change member,
   wherein the light concentration change member has a structure configured to concentrate, at one point, light rays transferred from the first optical waveguides in different positions are concentrated at one point.

2. The optical sensor of claim 1, wherein the light sources comprise laser diodes or light-emitting diodes.

3. The optical sensor of claim 1, wherein the light-emitting unit further comprises filters configured to filter in a specific waveband only, in case that the light sources are laser diodes.

4. The optical sensor of claim 1, wherein the light concentration change member comprises multiple grating couplers.

5. The optical sensor of claim 1, wherein the light concentration change member has a structure including at least one of a grating coupler, a lens, a slit, or a mirror and is configured to radiate light at a predetermined radiation angle with respect to the second direction.

6. The optical sensor of claim 4, wherein the multiple grating couplers are disposed in a lattice structure so as to change a radiation angle of first light transferred through a first path and second light transferred through a second path.

7. The optical sensor of claim 4, wherein the multiple grating couplers comprise rod-shaped structures.

8. The optical sensor of claim 4, wherein the multiple grating couplers are spaced apart by a predetermined distance in a parallel matrix and are arranged such that a longitudinal end of a grating coupler of the multiple grating couplers and a longitudinal front end of another grating coupler of the multiple grating couplers intersect with each other.

9. The optical sensor of claim 1, wherein the substrate comprises a printed circuit board (PCB) substrate or a photonic integrated circuit (PIC) substrate.

10. The optical sensor of claim 4, further comprising a lens structure configured to concentrate, at the one point, light rays discharged through the multiple grating couplers at a predetermined radiation angle with respect to the second direction .

11. An electronic device comprising an optical sensor comprising:

a light-emitting unit disposed on a substrate, the light-emitting unit comprising light sources configured to emit light in different wavebands;
first optical waveguides configured to transfer light emitted through the light-emitting unit in a first direction parallel to a surface of the substrate; and
a light concentration change member configured to change an angle of light such that light transferred from the first optical waveguides is discharged in a second direction substantially perpendicular to the first direction by the light concentration change member.

12. The electronic device of claim 11, wherein the light concentration change member has a structure configured to concentrate, at one point, light rays transferred in different positions.

13. The electronic device of claim 11, wherein the light sources comprise laser diodes or light-emitting diodes.

14. The electronic device of claim 11, wherein the light-emitting unit further comprises filters configured to filter in a specific waveband only, in case that the light sources are laser diodes.

15. The electronic device of claim 11, wherein the light concentration change member comprises multiple grating couplers.

# FIG. 1

EP 4 691 348 A1

FIG. 2

211    213    210    217

Optical sensor

| Light-emitting unit | Light concentration change member | Light-receiving unit |

FIG. 3A

# FIG. 3B

FIG. 4A

# FIG. 4B

450

FIG. 4C

<404>

# FIG. 5

FIG. 6

# FIG. 7A

FIG. 7B

FIG. 8A

FIG. 8B

# FIG. 9A

# FIG. 9B

FIG. 9C

# FIG. 10A

EP 4 691 348 A1

# FIG. 10B

210

EP 4 691 348 A1

33

1070    1060

1020  1030    1040    1050    1055  1045    1035  1025

1013

210

Z
↑
|
↓
-Z

<1003>

FIG. 10C

FIG. 11A

EP 4 691 348 A1

FIG. 11B

1160
1170
1115
1195
1191
1180
1110
210
1150
1140
1130
1120
z
-z

<1103>

<1104>

FIG. 11C

EP 4 691 348 A1

# FIG. 12

EP 4 691 348 A1

# FIG. 13A

1315

1310a

1310

1333

1310b

1310b

1330

L1

1335

1320

1350

L

<1301>

# FIG. 13B

1315

1310a

1310

1333

1310b

1310b

1330

1350

1320

<1302>

FIG. 13C

1315

1310a

1310

1333

1310b

1330

1350

1320

<1303>

# FIG. 14A

<u>1400</u>

1430

1432    1431

1420    1410

<1401>

1420    1410

<1402>

EP 4 691 348 A1

FIG. 14B

<1403>

<1404>

EP 4 691 348 A1

43

# FIG. 14C

1440

1450

1400

## INTERNATIONAL SEARCH REPORT

| International application No. |
| --- |
| **PCT/KR2024/005630** |

| A. | CLASSIFICATION OF SUBJECT MATTER |
| --- | --- |

**A61B 5/00**(2006.01)i

According to International Patent Classification (IPC) or to both national classification and IPC

| B. | FIELDS SEARCHED |
| --- | --- |

Minimum documentation searched (classification system followed by classification symbols)

A61B 5/00(2006.01); G01D 5/353(2006.01); G01J 3/18(2006.01); G01M 11/00(2006.01); G01N 21/00(2006.01); G01N 21/27(2006.01); G02B 6/00(2006.01); G02B 6/26(2006.01); G02B 6/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 광학 센서(optical sensor), 도파로(waveguide), 그레이팅 커플러(grating coupler), 회절(diffraction)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2017-0004607 A (SAMSUNG ELECTRONICS CO., LTD.) 11 January 2017 (2017-01-11) See paragraphs [0054]-[0063], [0074] and [0079]; claims 1 and 13; and figures 1-11. | 1-15 |
| Y | KR 10-2022-0082268 A (ELECTRONICS AND TELECOMMUNICATIONS RESEARCH INSTITUTE) 17 June 2022 (2022-06-17) See paragraphs [0044]-[0049]; claim 1; and figures 1-8. | 1-15 |
| A | KR 10-2015-0146468 A (GWANGJU INSTITUTE OF SCIENCE AND TECHNOLOGY) 31 December 2015 (2015-12-31) See paragraphs [0028]-[0084]; and figures 4a-4b. | 1-15 |
| A | KR 10-2008-0036909 A (CHUNG, Kyoung Hie et al.) 29 April 2008 (2008-04-29) See paragraphs [0090]-[0152]; and figures 1-2. | 1-15 |
| A | US 2010-0302544 A1 (DUER, Reuven) 02 December 2010 (2010-12-02) See paragraphs [0012]-[0050]. | 1-15 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **26 July 2024** | **26 July 2024** |

| Name and mailing address of the ISA/KR | Authorized officer |
| --- | --- |
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

| International application No. |
|---|
| **PCT/KR2024/005630** |

| Patent document cited in search report | | | Publication date (day/month/year) | Patent family member(s) | | | Publication date (day/month/year) |
|---|---|---|---|---|---|---|---|
| KR | 10-2017-0004607 | A | 11 January 2017 | CN | 106308776 | A | 11 January 2017 |
| | | | | CN | 117379046 | A | 12 January 2024 |
| | | | | EP | 3111834 | A1 | 04 January 2017 |
| | | | | EP | 3111834 | B1 | 07 July 2021 |
| | | | | KR | 10-2434698 | B1 | 22 August 2022 |
| | | | | US | 10420470 | B2 | 24 September 2019 |
| | | | | US | 11229363 | B2 | 25 January 2022 |
| | | | | US | 2017-0000350 | A1 | 05 January 2017 |
| | | | | US | 2019-0365231 | A1 | 05 December 2019 |
| KR | 10-2022-0082268 | A | 17 June 2022 | | None | | |
| KR | 10-2015-0146468 | A | 31 December 2015 | CN | 106415220 | A | 15 February 2017 |
| | | | | CN | 106415220 | B | 29 January 2019 |
| | | | | KR | 10-1816675 | B1 | 11 January 2018 |
| | | | | US | 10473492 | B2 | 12 November 2019 |
| | | | | US | 2017-0205256 | A1 | 20 July 2017 |
| | | | | WO | 2015-199424 | A1 | 30 December 2015 |
| KR | 10-2008-0036909 | A | 29 April 2008 | CN | 101548214 | A | 30 September 2009 |
| | | | | JP | 2011-512641 | A | 21 April 2011 |
| | | | | KR | 10-0889976 | B1 | 24 March 2009 |
| | | | | US | 2010-0078546 | A1 | 01 April 2010 |
| | | | | WO | 2008-050969 | A1 | 02 May 2008 |
| US | 2010-0302544 | A1 | 02 December 2010 | CN | 102460254 | A | 16 May 2012 |
| | | | | CN | 102460254 | B | 06 May 2015 |
| | | | | CN | 107003234 | A | 01 August 2017 |
| | | | | CN | 107003234 | B | 21 January 2020 |
| | | | | EP | 2425286 | A1 | 07 March 2012 |
| | | | | EP | 2425286 | B1 | 24 June 2020 |
| | | | | EP | 3175222 | A1 | 07 June 2017 |
| | | | | EP | 3175222 | A4 | 04 April 2018 |
| | | | | EP | 3175222 | B1 | 21 August 2019 |
| | | | | JP | 2012-525595 | A | 22 October 2012 |
| | | | | JP | 2017-526912 | A | 14 September 2017 |
| | | | | JP | 5757535 | B2 | 29 July 2015 |
| | | | | JP | 6516828 | B2 | 22 May 2019 |
| | | | | KR | 10-2012-0035912 | A | 16 April 2012 |
| | | | | US | 10018566 | B2 | 10 July 2018 |
| | | | | US | 10551318 | B2 | 04 February 2020 |
| | | | | US | 10590493 | B2 | 17 March 2020 |
| | | | | US | 2007-0211985 | A1 | 13 September 2007 |
| | | | | US | 2009-0068668 | A1 | 12 March 2009 |
| | | | | US | 2011-0249260 | A1 | 13 October 2011 |
| | | | | US | 2012-0231532 | A1 | 13 September 2012 |
| | | | | US | 2013-0071850 | A1 | 21 March 2013 |
| | | | | US | 2014-0178861 | A1 | 26 June 2014 |
| | | | | US | 2016-0033412 | A1 | 04 February 2016 |
| | | | | US | 2017-0023477 | A1 | 26 January 2017 |
| | | | | US | 2017-0067829 | A1 | 09 March 2017 |
| | | | | US | 2017-0082617 | A1 | 23 March 2017 |
| | | | | US | 2018-0265937 | A1 | 20 September 2018 |
| | | | | US | 7951583 | B2 | 31 May 2011 |

Form PCT/ISA/210 (patent family annex) (July 2022)

**INTERNATIONAL SEARCH REPORT**
Information on patent family members

International application No.

**PCT/KR2024/005630**

| Patent document cited in search report | Publication date (day/month/year) | Patent family member(s) | | | Publication date (day/month/year) |
|---|---|---|---|---|---|
| | | US | 8187866 | B2 | 29 May 2012 |
| | | US | 8288157 | B2 | 16 October 2012 |
| | | US | 8675199 | B2 | 18 March 2014 |
| | | US | 9423397 | B2 | 23 August 2016 |
| | | US | 9528939 | B2 | 27 December 2016 |
| | | US | 9976192 | B2 | 22 May 2018 |
| | | WO | 2007-106382 | A2 | 20 September 2007 |
| | | WO | 2007-106382 | A3 | 12 June 2008 |
| | | WO | 2010-127001 | A1 | 04 November 2010 |
| | | WO | 2015-131056 | A2 | 03 September 2015 |
| | | WO | 2015-131056 | A3 | 19 November 2015 |
| | | WO | 2016-019026 | A1 | 04 February 2016 |
| | | WO | 2017-059425 | A1 | 06 April 2017 |

Form PCT/ISA/210 (patent family annex) (July 2022)